**Europäisches Patentamt**

**European Patent Office**

**Office européen des brevets**

(19)

(11) Veröffentlichungsnummer: **0 222 237
B1**

(12)

# EUROPÄISCHE PATENTSCHRIFT

(45) Veröffentlichungstag der Patentschrift:
**01.03.89**

(51) Int. Cl.⁴: **C07C 143/12, C07C 139/14**

(21) Anmeldenummer: **86114852.6**

(22) Anmeldetag: **25.10.86**

(54) Verfahren zur Herstellung beweglicher Pasten waschaktiver alpha-Sulfofettsäureestersalze hohen Feststoffgehalts.

(30) Priorität: **02.11.85 DE 3538910**

(43) Veröffentlichungstag der Anmeldung:
**20.05.87 Patentblatt 87/21**

(45) Bekanntmachung des Hinweises auf die Patenterteilung:
**01.03.89 Patentblatt 89/9**

(84) Benannte Vertragsstaaten:
**AT BE CH DE FR IT LI NL SE**

(56) Entgegenhaltungen:
**DE-A- 3 123 681
DE-A- 3 334 517
DE-A- 3 432 324
DE-B- 1 418 887**

(73) Patentinhaber: **Henkel Kommanditgesellschaft auf Aktien, Postfach 1100 Henkelstrasse 67,
D-4000 Düsseldorf-Holthausen(DE)**

(72) Erfinder: **Piorr, Robert, Dr., Kieselei 12,
D-4030 Ratingen-Hösel(DE)**
Erfinder: **Rommerskirchen, Hans-Josef, Saganerweg 21,
D-4000 Düsseldorf 12(DE)**
Erfinder: **Ritterbex, Horst, Beedstrasse 44,
D-4000 Düsseldorf 30(DE)**
Erfinder: **Jost, Frantisek, Dr., Bonner Strasse 14,
D-4000 Düsseldorf 13(DE)**

**Beschreibung**

Alpha-Sulfofettsäureestersalze erhält man bekanntlich in Form wäßriger Pasten durch Neutralisation von Alpha-Sulfofettsäureestern mit insbesondere wäßrigem Alkalihydroxid. Als technisches Ausgangsmaterial dienen Fette und/oder Öle natürlichen Ursprungs, die durch Esterspaltung und nachfolgende Veresterung mit niederen Alkanolen, insbesondere Methanol beziehungsweise durch Umesterung der natürlichen Triglyceride mit den niederen Alkanolen erhalten werden. Die anfallenden Fettsäureestergemische enthalten – je nach Ursprung des natürlichen Rohstoffs – Fettsäuren eines vergleichsweise breiten Bereichs, der üblicherweise durch $C_{10-24}$ abgedeckt ist. Die Sulfonierung dieser Fettsäureestergemische, insbesondere mit gasförmigem $SO_3$, führt zu mehr oder weniger stark verfärbten Rohsulfonsäuren, die gebleicht und durch Neutralisation auf den pH-Bereich von etwa 6 bis 7 in Estersulfonatpasten überführt werden müssen. In dieser Form haben sie heute zunehmende praktische Bedeutung als oberflächenaktive Mittel beziehungsweise Netzmittel für Wasch- und Reinigungsmittel aus nachwachsenden Rohstoffen natürlichen Ursprungs.

Eine besondere Schwierigkeit in der technischen Handhabung solcher Pasten von Alkalisalzen alpha-sulfonierter Fettsäurealkylester – im folgenden als Estersulfonatsalze bezeichnet – liegt in ihrem Konzentrations-/Viskositäts-Verhalten. Technisch anfallende Estersulfonatsalze bilden in wäßriger Abmischung nur bei vergleichsweise niedrigen Feststoffkonzentrationen – beispielsweise bis zu Feststoffgehalten von etwa 35 Gewichtsprozent – hinreichend bewegliche Lösungen beziehungsweise Suspensionen, um den störungsfreien Ablauf technischer Vorgänge zu gewährleisten. Bei höheren Estersulfonatsalzgehalten – Feststoffgehalte von etwa 40 Gewichtsprozent oder mehr – steigt die Viskosität der wäßrigen Zubereitung so stark an, daß ihre freie Beweglichkeit nicht mehr besteht. Hieraus resultieren schwerwiegende Einschränkungen: Der Versuch, unmittelbar hochkonzentrierte Estersulfonatsalzpasten durch Neutralisation des Rohsulfonsäuregemisches mit konzentrierter Alkalihydroxidlösung zu gewinnen, mißlingt, weil die Rührbarkeit und damit die gleichmäßige Vermischbarkeit des Reaktionsgemisches verloren geht. Gleichzeitig damit wird der Abtransport der Neutralisationswärme unmöglich. Durch lokale Konzentrations- und Temperaturspitzen treten unerwünschte Nebenreaktionen – insbesondere eine unerwünscht hohe Bildung von Disalzen der alpha-Sulfofettsäuren unter Esterspaltung – ein. Nachteilig ist begreiflicherweise auch, daß durch Viskositätsanstieg immobilisierte Estersulfonatpasten im großtechnischen Betrieb nicht mehr pumpbar sind. Es tritt der Verschluß von Rohrleitungen und damit eine nachhaltige Störung des Betriebs der Gesamtanlage ein.

Der Stand der Technik zu Estersulfonatsalzpasten der hier betroffenen Art beschäftigt sich intensiv mit diesen Besonderheiten. Vorgeschlagen wurde insbesondere, zur Verbesserung des Fließverhaltens von wäßrigen technischen Konzentraten von alpha-Sulfofettsäureestersalzen Fließhilfsmittel beziehungsweise Viskositätsregler einzusetzen. So schildert beispielsweise die DE-OS 3 305 430 den Einsatz von $C_{8-40}$-Alkoholen als Viskositätsregler, die zusätzlich eine oder mehrere Hydroxylgruppen als Substituenten aufweisen können und an die bis zu 20 Mol Ethylenoxid und/oder Propylenoxid pro Mol Alkanol angelagert sein können. Diese Viskositätsregler werden der wäßrigen Estersulfonatpaste in Mengen von 1 bis 15 Gewichtsprozent, bezogen auf die Tensidmenge, zugefügt, wobei sich eine Viskosität des Tensidkonzentrates von höchstens 10 000 mPas bei 70°C einstellt.

Die zuvor erwähnten Disalze der alpha-Sulfofettsäuren sind aus mehreren Gründen unerwünscht: Sie besitzen nur beschränkte Wasserlöslichkeit und zeigen darüber hinaus schlechte Oberflächenaktivitäten. Insbesondere beeinflussen sie aber auch die Viskosität der letztlich gewünschten wäßrigen Estersulfonatpasten. Ein zu hoher Gehalt führt zu einer beträchtlichen Viskositätssteigerung der wäßrigen Estersulfonatpasten.

Insbesondere in jüngster Zeit ist diesem Teilaspekt der insgesamt mit zahlreichen Schwierigkeiten verbundenen Gewinnung von Tensiden auf Basis von Estersulfonaten besondere Beachtung gewidmet worden. Verwiesen wird auf DE-OS 3 123 681 und DE-OS 3 334 517. Gemäß der zuerst genannten Druckschrift wird vorgeschlagen, zur Herstellung einer hochkonzentrierten wäßrigen Lösung eines Salzes von alpha-Sulfofettsäureestern das sulfonierte Fettsäureprodukt zweistufig mit einer wäßrigen Alkalilösung derart zu neutralisieren, daß in einer ersten Neutralisationsstufe mit einer höherkonzentrierten wäßrigen Alkalilösung (15 bis 50 Gewichtsprozent Alkali) in Gegenwart eines Alkohols mit 1 bis 4 Kohlenstoffatomen in einer Menge von 5 bis 20 Gewichtsprozent – bezogen auf das Gewicht des sulfonierten Produkts – bis auf einen pH-Wert 2,5 bis 4 neutralisiert wird, woraufhin in einer anschließenden Neutralisationsstufe mit einer stärker verdünnten wäßrigen Alkalilösung auf einen pH-Wert von 6 bis 7 zu Ende neutralisiert wird. Vor dieser zweistufigen Neutralisation kann das Sulfonierungsrohprodukt auch gebleicht werden. Bevorzugt wird hierzu eine wäßrige Lösung von $H_2O_2$ ebenfalls in Gegenwart eines Alkohols mit 1 bis 4 Kohlenstoffatomen eingesetzt. Das Wasserstoffperoxid soll in Form einer wäßrigen Lösung mit einer Konzentration von 10 Gewichtsprozent oder mehr verwendet werden. Der bevorzugte Alkohol ist Methanol, wenn es sich bei den Fettsäureestern um Methylester handelt. Nach diesem Vorschlag soll es möglich sein, den Disalzgehalt der entsprechenden alpha-Sulfofettsäuren auf 5% oder weniger zurückzudrängen.

Die DE-OS 3 334 517 beschreibt dann jedoch die Nachteile dieses Vorschlages: Die Sulfonierungsprodukte enthalten den in beträchtlichem Überschuß eingesetzten kurzkettigen Alkohol im wäßrigen neutralisierten Reaktionsgut in hohen Mengen. Das ist unerwünscht. Der kurzkettige Alkohol stört bei der Herstellung von Waschmittelgemischen durch Sprühtrocknung, insbesondere wird dabei unerwünschtes "Pluming" ausgelöst. Die im Tensidgemisch vorliegenden freien Alkohole weisen darüber hinaus einen unerwünschten Fremdgeruch auf, so daß eine Desodorierung erforderlich ist. Zur Problemlösung schlägt die zuletzt genannte Druckschrift vor, die wäßrige Bleiche und die Neutralisation der rohen alpha-Sulfofettsäureester in Gegenwart solcher Mengen eines niedrigen Alkohols vorzunehmen, daß eine wäßrige Aufschlämmung mit 30 bis 55 Gewichtsprozent des alpha-Sulfofettsäureestersalzes und – bezogen auf das Gewicht des alpha-Sulfofettsäureestersalzes – 5 bis 15 Gewichtsprozent eines niedrigen Alkoholsulfats und 8 bis 40 Gewichtsprozent des niedrigen Alkohols erhalten wird. Abschließend soll die wäßrige Aufschlämmung derart eingeengt werden, daß sie 40 bis 65 Gewichtsprozent an alpha-Sulfofettsäureestersalz, 2 bis 10 Gewichtsprozent eines niedrigen Alkoholsulfats und gegebenenfalls höchstens 2 Gewichtsprozent eines niedrigen Alkohols enthält.

Die Lehre der älteren Schutzrechtsanmeldung P 34 32 324.4 (D 7116) geht von der überraschenden Feststellung aus, daß durch eine sehr viel zweckmäßigere Maßnahme die unerwünschte Bildung von alpha-Sulfofettsäure-Disalzen verhindert werden kann. Das Arbeiten mit großen Alkoholüberschüssen und das gegebenenfalls anschließende Eindampfen zur Beseitigung unerwünschter Alkoholanteile entfällt. Dieses Verfahren baut auf der Erkenntnis auf, daß eine gezielt bemessene Behandlung der Rohsulfonsäure von Fettsäurealkylestern mit beliebigen Alkoholen in geringer, jedoch im folgenden definierter Menge zur regelbaren Senkung des Gehalts an unerwünschtem Disalz führt, sofern diese Nachreaktion vor einer nachfolgenden Behandlung mit wäßrigen Medien vorgenommen wird. Eine weitere wichtige Voraussetzung für den Ablauf der Nachreaktion im gewünschten Sinne ist eine hinreichend hohe Reaktionstemperatur, die bevorzugt oberhalb von 60°C und insbesondere oberhalb von 75°C liegt. Als Ergebnis dieser Nachreaktion der Rohsulfonsäure mit Alkoholen vor einer Einführung von Wasser in das Reaktionsgemisch tritt unter anderem die Bildung von Estern der alpha-sulfonierten Fettsäuren mit den nachträglich in die Reaktionsmischung eingeführten Alkohole ein. Das Reaktionsprodukt läßt sich damit als partiell umgeestertes Gemisch von alpha-Sulfofettsäureestern beschreiben, das nach seiner wäßrigen Aufarbeitung letztlich weitere Komponenten – unter anderem auch beschränkte Mengen an Alkoholsulfaten – enthält.

Die Lehre der Erfindung geht von der überraschenden Feststellung aus, daß Reaktionsgemische aus der genannten älteren Anmeldung dazu befähigt sind, nach ihrer Aufbereitung und Umwandlung in Estersulfonatsalzpasten besonders erwünschte Konzentrations-/Viskositäts-Verhältnisse zu liefern. Es hat sich überraschenderweise gezeigt, daß es mit Hilfe der geschilderten Nachreaktion möglich wird, letztlich Disalz-arme Estersulfonatpasten herzustellen, die bei Feststoffgehalten oberhalb 35 Gewichtsprozent und insbesondere im Bereich von etwa 40 bis 65 Gewichtsprozent an waschaktiver Substanz in wäßriger Lösung beziehungsweise Aufschlämmung auch bei nur mäßig erhöhten Temperaturen bewegliche und insbesondere pumpfähige Stoffgemische darstellen.

Die zur Nachreaktion eingesetzte bestimmte Alkoholkomponente beeinflußt dabei dieses Viskositäts-/Konzentrations-Verhalten, so daß es erfindungsgemäß möglich wird, hochkonzentrierte fließfähige Lösungen beziehungsweise Pasten wäßriger Estersulfonatsalze herzustellen, die beispielsweise in Tankwagen transportiert und daraus verarbeitet werden können.

Gegenstand der Erfindung ist dementsprechend in der bevorzugten Ausführungsform ein Verfahren zur Herstellung von wäßrigen Pasten waschaktiver alpha-Sulfofettsäureestersalze, die bei hohen Fettstoffgehalten auch bei nur mäßig erhöhten Temperaturen beweglich, insbesondere pumpfähig sind, durch Sulfonieren von Fettsäurealkylestern mit $SO_3$ in Molverhältnissen bis 1 : 2, insbesondere im Bereich von 1 : 1,2 bis 1,8, und nachfolgende Aufarbeitung der Rohsulfonsäure in wäßrigem Medium unter Salzbildung, wobei dieses Verfahren dadurch gekennzeichnet ist, daß man die Rohsulfonsäure vor der Behandlung mit einem wäßrigen Medium einer Nachreaktion mit wenigstens etwa 0,5 Mol-Äquivalent – bezogen auf den zur alpha-Sulfonierung nicht verbrauchten $SO_3$-Anteil – an 1-wertigen Alkoholen und/oder deren Alkoxylierungsprodukten bei Temperaturen oberhalb 70°C unterwirft und in der nachfolgenden wäßrigen Aufarbeitung Feststoffgehalte der alpha-Sulfofettsäureestersalze oberhalb 35 Gewichtsprozent einstellt.

Der Feststoffgehalt der erfindungsgemäß hergestellten Estersulfonatsalzpasten und insbesondere der Gehalt an dem entstehenden Estergemisch der alpha-sulfonierten Fettsäuren liegt bevorzugt im Bereich von etwa 40 bis 65 Gewichtsprozent.

Zur Nachreaktion der Rohsulfonsäure vor dessen Behandlung mit wäßrigen Medien sind insbesondere monofunktionelle Alkanole beziehungsweise deren Alkoxylierungsprodukte mit bis zu 30 C-Atomen im Alkoholrest geeignet, wobei im Falle der entsprechenden Alkoxylierungsprodukte vorzugsweise bis zu 20 Alkoxyreste im Molekül vorliegen können. Besonders geeignet sind entsprechend Hydroxylgruppen enthaltende Komponenten mit 2 bis 22 C-Atomen in der Alkoholkomponente. Bevorzugt ist es weiterhin, beim Arbeiten mit Alkoxylierungsprodukten dieser Alkohole entsprechende Verbindungen einzusetzen, die wenigstens anteilsweise vorzugsweise zu wenigstens 50% EO-Gruppen – das heißt Gruppen der Konfiguration –$CH_2CH_2O$– – enthalten. In an sich bekannter Weise können neben solchen EO-Gruppierungen – gewünschtenfalls aber auch an ihrer Stelle – entsprechende Reste vorliegen, die sich vom Propylenoxid ableiten.

Eine besonders interessante Verbindungsklasse zur Einstellung der erfindungsgemäß gewünschten günstigen Viskositäts-/Konzentrations-Verhältnisse sind Alkoxylierungsprodukte niederer Alkohole mit insbesondere bis zu 6 C-Atomen, vorzugsweise mit 2 bis 4 C-Atomen im Alkoholrest. Solche Alkoxylierungsprodukte können beispielsweise 2 bis 15, vorzugsweise 5 bis 12 EO-Einheiten im Alkylenglykoletherrest enthalten. Die zur Nachreaktion eingesetzte ethoxylierte Alkoholkomponente ist damit als solche hinreichend hochsiedend, so daß im Reaktionsgemisch verbleibende Restgehalte dieser Komponente bei der weiteren Aufarbeitung der Estersulfonatsalze nicht stören. Darüber hinaus erweisen sich diese Komponenten aber auch als besonders wirksam in der Viskositätssenkung hochkonzentrierter wäßriger Estersulfonatpasten. So kann beispielsweise durch die Nachreaktion der Rohsulfonsäure mit einem Ethanol/10 EO-Kondensat zu wäßrigen Lösungen führen, die selbst bei Feststoffgehalten von 60 Gewichtsprozent und darüber im Temperaturbereich von beispielsweise 40 bis 60° vergleichsweise dünnflüssige Lösungen sind und sich dementsprechend für den störungsfreien Tankwagentransport eignen.

Aber auch die Verwendung von typischen Fettalkoholen, das heißt Alkoholen des C-Zahlbereichs von etwa 10 bis 20, und/oder deren Alkoxylierungsprodukten führt zu beweglichen Pasten, die beispielsweise noch bei Feststoffgehalten im Bereich von 60 Gewichtsprozent und Temperaturen von 50 bis 60° pumpfähig sind.

Ein besonderes Kennzeichen dieser erfindungsgemäß hergestellten Estersulfonatpasten liegt darin, daß sich die angestrebten günstigen Viskositäts-/Konzentrations-Verhältnisse über den gesamten Konzentrationsbereich von etwa 35 bis 65 Gewichtsprozent Feststoff nicht substanziell ändern. Hier liegt gegenüber den ohne Einschaltung der erfindungsgemäß vorgesehenen alkoholischen Nachreaktion hergestellten Fettsäuremethylestersulfonatsalze eine entscheidende Abweichung. In der ebenfalls nicht vorveröffentlichten älteren Schutzrechtsanmeldung P 34 39 520.2 (D 7074) sind bei Temperaturen von wenigstens 60°C wäßrige Pasten von Alkalisalzen alpha-sulfonierter Fettsäurealkylester beschrieben, deren Estersulfonatsalz-Feststoffgehalte wenigstens etwa 60 Gewichtsprozent beträgt und die dabei praktisch frei von Viskositätsreglern sind. Wesentlich dafür ist aber die Einschränkung der Fettsäurebasis auf $C_{16}$ und/oder $C_{18}$-Fettsäuren. Nur bei der Auswahl dieser Fettsäuren wird im Bereich sehr hoher Feststoffgehalte der Estersulfonatpasten, der bei 60 bis 70 Gewichtsprozent liegt, die gewünschte Viskositätserniedrigung erreicht. Bei niedrigeren Konzentrationen entsprechender Estersulfonate – insbesondere im Bereich von 40 bis 55 Gewichtsprozent durchlaufen die entsprechenden wäßrigen Aufschlämmungen hochviskose Phasen, so daß bei der kontinuierlichen Herstellung solcher hochkonzentrierter Estersulfonatpasten schwierige und vergleichsweise komplizierte Verfahrensschritte zu durchlaufen sind, vergleiche hierzu im einzelnen die Angaben der genannten älteren Schutzrechtsanmeldung.

Die der erfindungsgemäßen alkoholischen Nachreaktion unterworfenen Produkte sind demgegenüber ohne Schwierigkeiten auch und gerade im kritischen Konzentrationsbereich von etwa 35 bis 60 Gewichtsprozent Feststoff leicht zu bewegen und damit leicht zu verarbeiten.

Zu den Verfahrensbedingungen der Nachreaktion im einzelnen gelten weitgehend die Angaben der genannten älteren Schutzrechtsanmeldung (DE-Patentanmeldung 34 32 324.4). Es gilt damit das Folgende:

In einer bevorzugten Ausführungsform des erfindungsgemäßen Verfahrens wird die Einschränkung des Gehalts an freiem Alkohol im Reaktionsprodukt vorgesehen. Hierzu werden in der Umesterungsstufe nicht mehr als etwa 2-Mol-Äquivalent der freien Alkoholkomponente eingesetzt. Insbesondere wird in dieser Stufe mit nicht mehr als etwa 1,5 Mol-Äquivalent des Alkohols gearbeitet. Hierbei beziehen sich diese Mol-Äquivalentangaben jeweils wieder auf den im Sulfonierungsrohprodukt vorliegenden $SO_3$-Anteil, der nicht zur alpha-Sulfonierung verbraucht worden ist. Diese $SO_3$-Bezugsbasis errechnet sich damit als Summe von 2 Teilbeträgen. Der eine Teilbetrag entspricht dem $SO_3$-Überschuß, der in der Sulfonierungsstufe – zur Steigerung des Umsetzungsgrades – über die zur alpha-Sulfonierung benötigte $SO_3$-Menge hinaus eingesetzt worden ist. Der andere Teilbetrag errechnet sich als Differenz der theoretisch benötigten $SO_3$-Menge abzüglich des in der alpha-Sulfonierung tatsächlich verbrauchten Anteils.

Allgemein gilt, daß Mengen nicht über etwa 1,3 Mol-Äquivalent des Alkohols – bezogen auf die zuvor erläuterte $SO_3$-Menge – zweckmäßig sein können, wobei das Arbeiten mit Alkoholmengen im Bereich von 0,8 bis 1,3 Mol-Äquivalent Alkohol und insbesondere 0,9 bis 1,1 Mol-Äquivalent Alkohol besonders bevorzugt sein kann.

Die Bedingungen der Umesterungsreaktion werden – insbesondere unter Anpassung an die Reaktivität des zur Umesterung eingesetzten Alkohols – so gewählt, daß das Ausmaß der zusätzlichen thermischen Belastung des Reaktionsgemisches möglichst gering gehalten wird. Auf diese Weise kann die Bildung unerwünschter zusätzlicher Verfärbungen im Reaktionsprodukt eingeschränkt oder verhindert werden. Hinreichend intensive Reaktionsbedingungen sind allerdings notwendig, um die erfindungsgemäß geforderte Umesterung zu bewirken. Allgemein gilt, daß die Nachreaktion bei Temperaturen nicht oberhalb etwa 150°C, vorzugsweise bei Temperaturen nicht über 120°C, durchgeführt wird. Besonders geeignet können Temperaturen oberhalb 75°C sein. Ein geeigneter Temperaturbereich ist beispielsweise 80°C bis 100°C. Die Reaktionsdauer wird in Abhängigkeit von der Reaktivität des zur Umesterung eingesetzten Alkohols und der gewählten Reaktionstemperatur bestimmt. Im allgemeinen werden wenigstens 5, insbesondere wenigstens 10 Minuten Reaktionszeit erforderlich sein, geeignet ist üblicherweise der Bereich von etwa 10 bis 30 Minuten.

Zur Auswahl der Verfahrensbedingungen im einzelnen gilt die folgende allgemeine Gesetzmäßigkeit: In der Umesterungsreaktion vergleichsweise hochreaktiv sind niedere Alkohole. Höhere Alkohole zeigen im allgemeinen eine geringere Reaktionsfähigkeit. Als Beispiel seien hier Fett- oder Wachsalkohole genannt. Ihre Verwendung benötigt also innerhalb des genannten Rahmens intensivere Umsetzungsbedingungen.

Die Auswahl der Reaktionskomponenten und Arbeitsbedingungen wird im allgemeinen so getroffen, daß der Disalzgehalt im mit wäßrigen Medien behandelten und neutralisierten Reaktionsprodukt kleiner 10 Gewichtsprozent — bezogen auf Waschaktivsubstanz liegt. Bevorzugt werden Mengen des Disalzgehalts gleich oder kleiner 5 Gewichtsprozent, wobei es aber möglich ist, auch noch zu geringeren Werten, beispielsweise Mengen kleiner 2 Gewichtsprozent zu kommen.

Ganz allgemein gilt, daß die zur Umesterung eingesetzten Alkohole in einer bevorzugten Ausführungsform ihrerseits im Molekül neben der Hydroxylgruppe keine reaktiven Gruppen aufweisen, die zu unerwünschten Nebenreaktionen befähigt sind.

Als Alkoholkomponenten kommen beispielsweise in Frage: Monofunktionelle aliphatische und cycloaliphatische Alkohole mit 1 bis 30, vorzugsweise 1 bis 24 Kohlenstoffatomen wie Methanol, Ethanol, n-Propanol, i-Propanol, n-Butanol, 2-Butanol, n-Pentanol, 2-Pentanol, n-Hexanol, n-Octanol, n-Decanol, n-Dodecanol, n-Tetradecanol, n-Hexadecanol, n-Octadecanol, n-Eikosanol, n-Docosanol, 2-Hexyldecanol, 2-Octyldodecanol, 2-Dodecylhexadecanol, Oxoalkohole mit 9 bis 18 Kohlenstoffatomen, Ziegler-Alkohole mit 8 bis 20 Kohlenstoffatomen, Cyclohexanol und Methylcyclohexanole.

Weitere Beispiele für mögliche Alkoholkomponenten sind Glykolhalbether wie Methylethylenglykol, Ethylethylenglykol und Anlagerungsprodukte von 1 bis 20 Mol Ethylen- und/oder Propylenoxid an aliphatische Alkohole mit 1 bis 24 Kohlenstoffatomen, insbesondere an Fettalkohole und Fettalkoholgemische.

Die genannten Alkohole können im erfindungsgemäßen Verfahren einzeln oder im Gemisch eingesetzt werden. Fettalkohole im engeren Sinn, d.h. geradkettige aliphatische Alkohole mit 8 bis 24 Kohlenstoffatomen werden — ihrer Herkunft entsprechend — im Regelfall als Gemische eingesetzt, wobei die Zusammensetzung solcher Alkoholgemische von den natürlichen Fetten und Ölen bestimmt wird, die als Ausgangsmaterial für ihre Herstellung verwendet werden.

Die der Umesterung zu unterwerfende Rohsulfonsäure soll in einer bevorzugten Ausführungsform des erfindungsgemäßen Verfahrens einen in der alpha-Sulfonierung nicht verbrauchten $SO_3$-Anteil von nicht mehr als 80 Mol-% und vorzugsweise von nicht mehr als 50 Mol-% — jeweils bezogen auf den gebildeten alpha-Sulfofettsäureester — aufweisen. Bevorzugt ist es weiterhin, daß diese Rohsulfonsäure einen Sulfonierungsgrad von wenigstens 90%, insbesondere von wenigstens 95% und zweckmäßigerweise von 98% oder mehr — jeweils bezogen auf den eingesetzten Fettsäureester — aufweisen.

Für die der Umesterungsstufe vorgängige Sulfonierung gelten die Angaben des Standes der Technik, verwiesen sei beispielsweise auf die Angaben der DE-AS 1 246 718 und der DE-AS 1 248 645. Als Ausgangsmaterial für diese Sulfonierung werden bevorzugt niedere Alkylester, insbesondere die Methylester von Fettsäuren eingesetzt, die beispielsweise 6 bis 28 und vorzugsweise 8 bis 18 Kohlenstoffatome enthalten. Diese Fettsäureester entstammen bevorzugt natürlichen Fetten von Pflanzen, Land- oder Wassertieren. Sie sollen abgesehen vom alpha-ständigen Wasserstoffatom keine weiteren sulfatierbaren oder sulfonierbaren Gruppen, insbesondere Doppelbindungen oder alkoholische Hydroxylgruppen enthalten. Ihre Jodzahlen liegen unterhalb von 5, vorzugsweise unterhalb von 2. Die Sulfonierung erfolgt mit einem $SO_3$/Inertgas-Gemisch, das üblicherweise 2 bis 40 Volum-% $SO_3$ enthalten kann, bei Temperaturen, die 100°C und vorzugsweise 95°C nicht oder nicht wesentlich überschreiten. Es kann bei konstanter Verfahrenstemperatur oder mit stufenförmiger Temperaturführung gearbeitet werden wie es in den zuvor genannten Druckschriften beschrieben ist.

An die erfindungsgemäße Nachreaktion mit Alkoholen schließt sich die Aufarbeitung des Reaktionsprodukts mit wäßrigen Medien in an sich bekannter Weise an. Hierbei handelt es sich insbesondere um die Bleiche und die Neutralisation der erfindungsgemäß umgeesterten Rohsulfonsäure. Die Bleiche kann in an sich bekannter Weise mit wäßrigem Wasserstoffperoxid und/oder Hypochloritlösung erfolgen. Die Neutralisation kann der Bleiche vorgängig sein oder erst nach der Bleiche durchgeführt werden. Die saure Bleiche mit Wasserstoffperoxid ist beispielsweise in der DE-PS 1 179 931 beschrieben, eine Kombinationsbleiche, bei der sich an eine zunächst saure Peroxidbleiche die Neutralisierung des sulfonierten und teilgebleichten Gutes anschließt, woraufhin erneut mit Wasserstoffperoxid oder besser mit Hypochlorit eine abschließende Bleichstufe erfolgt, beschreibt die DE-AS 1 234 709.

Die Verfahrenbedingungen von Bleichstufe und/oder Neutralisation sind dabei so zu wählen, daß hier die prinzipiell mögliche Esterverseifung ausgeschlossen oder soweit wie möglich unterdrückt wird. Ohne diese Vorsichtsmaßnahmen würden die Vorteile der erfindungsgemäßen Umesterungsstufe bezüglich der Reduzierung des Disalzgehaltes wenigstens teilweise wieder verloren gehen.

Beispiele

Beispiel 1

In einem Fallfilmreaktor wurden 283 g (1 Mol) gehärteter Talgfettsäuremethylester (Jodzahl 0,5; Verseifungszahl 198) bei 90°C mit 96 g (1,2 Mol) Schwefeltrioxid (5 Vol.-% in Luft) sulfoniert. Das resultierende Reaktionsgemisch wurde anschließend 30 Minuten lang bei 90°C gealtert. Der Sulfonierungsgrad betrug danach 98%.

Das gealterte rohe Sulfonierungsprodukt wurde bei 90°C unter Rühren mit 7,0 g (0,22 Mol) Methanol versetzt und 20 Minuten lang bei 90°C weitergeführt. Anschließend wurde das Reaktionsprodukt zur Bleichung mit 16 g Wasserstoffperoxid in Form einer 35-gewichtsprozentigen wäßrigen Lösung versetzt und 10 Minuten lang bei 60°C gerührt, bevor es durch Zugabe einer 25-gewichtsprozentigen Natriumhydroxidlösung bis zum pH-Wert 7 neutralisiert wurde.

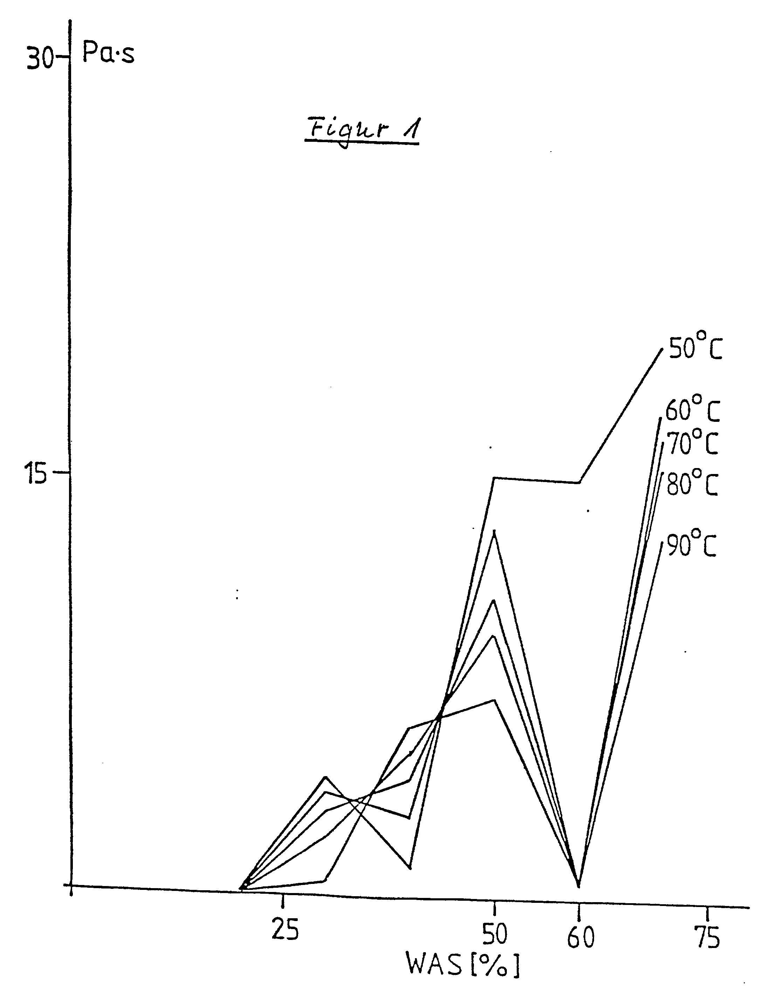

Teilmengen der erhaltenen konzentrierten alpha-Sulfofettsäureestersalzlösung wurden auf Waschaktivsubstanzgehalte von 30, 40, 50, 60 und 70 Gewichtsprozent eingestellt. Die scheinbaren Viskositäten der erhaltenen Lösungen wurden mit Hilfe eines Rotationsrheometers (Rheomat 30 in Kombination mit Meßsystemen vom Typ DIN 14 oder DIN 25 und einer Datenstation HP 85; Hersteller: Contraves AG, Zürich, Schweiz) bei 50, 60, 70, 80 und 90°C und einer Schergeschwindigkeit von 100 s⁻¹ gemessen. Die gefundenen Daten sind in Figur 1 graphisch dargestellt.

Beispiel 2

In einem Fallfilmreaktor wurden 283 g (1 Mol) gehärteter Talgfettsäuremethylester (Jodzahl 0,5; Verseifungszahl 198) bei 90°C mit 112 g (1,4 Mol) Schwefeltrioxid (5 Vol.-% in Luft) sulfoniert. Das dabei erhaltene Reaktionsgemisch wurde 20 Minuten lang bei 90°C gealtert. Der Sulfonierungsgrad betrug 98%.

Das gealterte rohe Sulfonierungsprodukt wurde bei 90°C unter Rühren mit 214 g (0,44 Mol) eines Anlagerungsproduktes von 10 Mol Ethylenoxid an Ethanol versetzt und 20 Minuten lang bei 90°C weitergerührt. Anschließend wurde das Reaktionsprodukt wie in Beispiel 1 mit Wasserstoffperoxid gebleicht und mit Natriumhydroxidlösung neutralisiert.

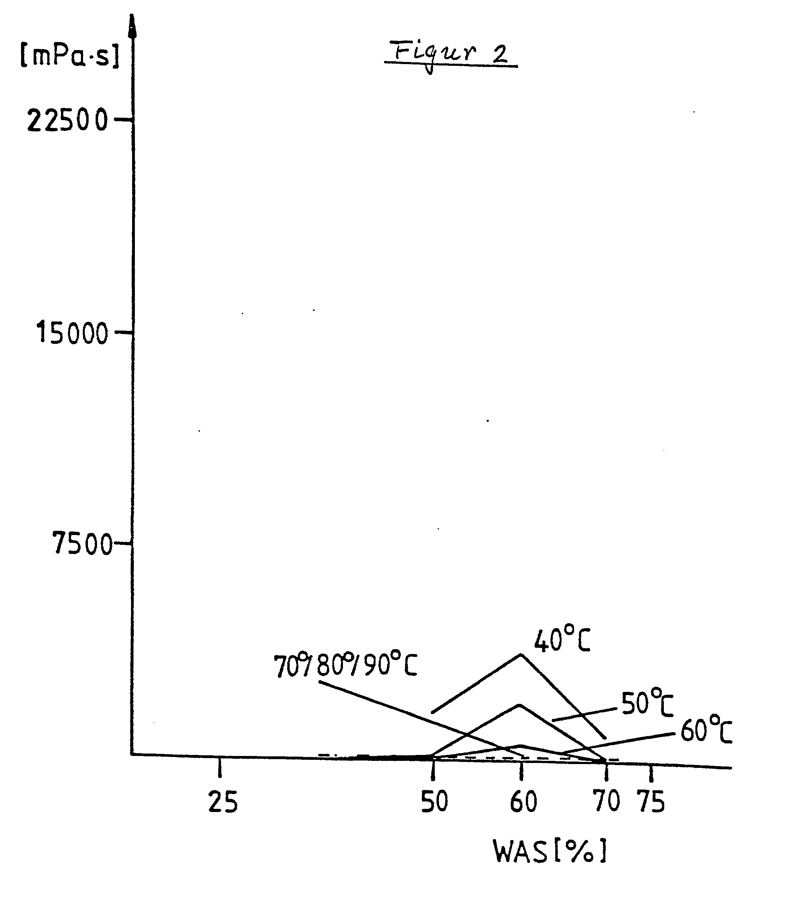

Teilmengen der erhaltenen konzentrierten alpha-Sulfofettsäureestersalzlösung wurden auf Waschaktivsubstanzgehalte von 40, 50, 60, und 70 Gewichtsprozent eingestellt. Die scheinbaren Viskositäten dieser Lösungen wurden – wie in Beispiel 1 beschrieben – bei 40, 50, 60, 70, 80 und 90°C und einer jeweiligen Schergeschwindigkeit von 100 s⁻¹ bestimmt. Die gefundenen Werte sind in Figur 2 graphisch dargestellt.

Beispiel 3

Zu Vergleichszwecken wurden 283 g (1 Mol) Talgfettsäuremethylester (Jodzahl 0,5; Verseifungszahl 198) bei 90°C mit 96 g (1,2 Mol) Schwefeltrioxid (5 Vol.-% in Luft) sulfoniert. Anschließend wurde das Reaktionsgemisch 30 Minuten lang bei 90°C gealtert. Der Sulfonierungsgrad betrug 98%.

Ohne vorausgehende Umsetzung mit einem Alkohol wurde das gealterte, rohe Sulfonierungsprodukt wie in Beispiel 1 gebleicht und neutralisiert.

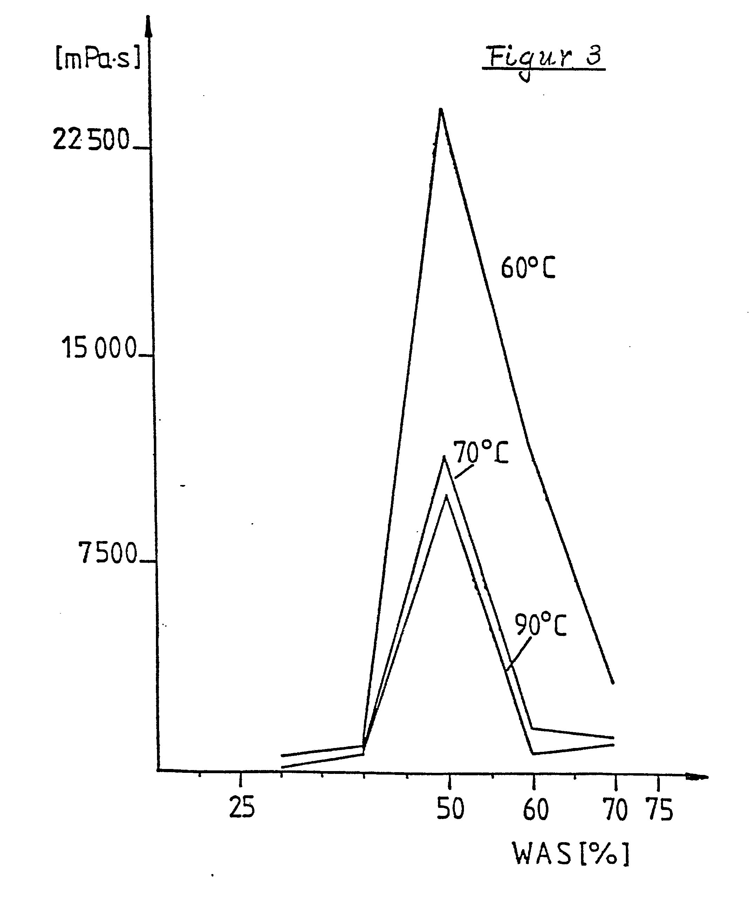

Teilmengen der erhaltenen konzentrierten alpha-Sulfofettsäureestersalzlösung wurden auf Waschaktivsubstanzgehalte von 30, 40, 50, 60 und 70 Gewichtsprozent eingestellt. Die scheinbaren Viskositäten dieser Lösungen wurden – wie in Beispiel 1 beschrieben – bei 60, 70 und 90°C und einer Schergeschwindigkeit von 100 s⁻¹ ermittelt. Die gefundenen Werte sind in Figur 3 graphisch dargestellt.

**Patentansprüche**

1. Verfahren zur Herstellung von wäßrigen Pasten waschaktiver alpha-Sulfofettsäureestersalze, die bei hohen Feststoffgehalten auch bei mäßig erhöhten Temperaturen beweglich, insbesondere pumpfähig sind, durch Sulfonieren von Fettsäurealkylestern mit SO₃ in Molverhältnissen bis 1 : 2, insbesondere von 1 : 1,2 bis 1,8, und nachfolgende Aufarbeitung der Rohsulfonsäure in wäßrigem Medium unter Salzbildung, dadurch gekennzeichnet, daß man die Rohsulfonsäure vor der Behandlung mit einem wäßrigen Medium einer Nachreaktion mit wenigstens etwa 0,5 Mol-Äquivalent – bezogen auf den zur alpha-Sulfonierung nicht verbrauchten SO₃-Anteil – an 1-wertigen Alkoholen und/oder deren Alkoxylierungsprodukten bei Temperaturen oberhalb 70°C unterwirft und in der nachfolgenden wäßrigen Aufarbeitung Feststoffgehalte der alpha-Sulfofettsäureestersalze oberhalb von 35 Gewichtsprozent einstellt.

2. Verfahren nach Anspruch 1, dadurch gekennzeichnet, daß man die Nachreaktion mit den Alkoholen beziehungsweise ihren Alkoxylierungsprodukten bei Temperaturen oberhalb 75°C, vorzugsweise im Bereich von 80 bis 100°C durchführt und dabei insbesondere etwa äquimolare Mengen der Hydroxylgruppen enthaltenden Komponenten – bezogen auf den zur alpha-Sulfonierung nicht verbrauchten SO₃-Anteil – einsetzt.

3. Verfahren nach Ansprüchen 1 und 2, dadurch gekennzeichnet, daß man bei der nachfolgenden wäßrigen Aufarbeitung – insbesondere der Bleiche und/oder der Neutralisation zum Estersulfonatsalz – Pastenfeststoffgehalte von etwa 40 bis 65 Gewichtsprozent einstellt.

4. Verfahren nach Ansprüchen 1 bis 3, dadurch gekennzeichnet, daß die Nachreaktion mit monofunktionellen Alkanolen beziehungsweise deren Alkoxylierungsprodukten mit bis zu 30 C-Atomen im Alkoholrest und vorzugsweise mit bis zu 20 Alkoxyresten im Molekül durchgeführt wird, wobei man bevorzugt mit C₂₋₂₂-Alkoholen und/oder deren Alkoxylierungsprodukten arbeitet, die wenigstens anteilsweise EO-Gruppen enthalten.

5. Verfahren nach Ansprüchen 1 bis 4, dadurch gekennzeichnet, daß man die Nachreaktion mit Alkoxylierungsprodukten niederer Alkohole mit bis zu 6 C-Atomen, vorzugsweise mit 2 bis 4 C-Atomen und 2 bis 15, vorzugsweise 5 bis 12 EO-Einheiten im Alkoxyrest und/oder mit Fettalkoholen beziehungsweise deren Alkoxylierungsprodukten durchführt.

6. Verfahren nach Ansprüchen 1 bis 5, dadurch gekennzeichnet, daß Rohsulfonsäure der Nachreaktion unterworfen werden, deren in der alpha-Sulfonierung nicht verbrauchter SO₃-Anteil nicht mehr als 80 Mol-%, vorzugsweise nicht mehr als 50 Mol-% – jeweils bezogen auf alpha-Sulfofettsäureester – beträgt, wobei diese Rohsulfonsäure bevorzugt einen Sulfonierungsgrad von wenigstens 90%, insbesondere von wenigstens 95% aufweisen.

7. Verfahren nach Ansprüchen 1 bis 6, dadurch gekennzeichnet, daß Estersulfonatpasten mit einem Di-Salzgehalt nicht über ca. 10 Gewichtsprozent, vorzugsweise nicht über 5 Gewichtsprozent – jeweils bezogen auf Waschaktivsubstanz – hergestellt werden, die vorzugsweise gleichzeitig im wesentlichen frei sind von ungebundenem Alkohol in störenden Mengen.

## Claims

1. A process for the production of aqueous pastes of tensio-active α-sulfofatty acid ester salts which, despite high solids contents, are mobile and, in particular, pumpable even at moderately elevated temperatures by sulfonation of fatty acid alkylesters with $SO_3$ in molar ratios of up to 1 : 2 and more especially of from 1 : 1.2 to 1 : 1.8 and subsequent working-up of the crude sulfonic acid in aqueous medium with salt formation, characterized in that, before the treatment with an aqueous medium, the crude sulfonic acid is subjected to an afterreaction with at least about 0.5 mole equivalent, based on the $SO_3$ not used for the α-sulfonation, of monohydric alcohols and/or alkoxylation products thereof at temperatures above 70°C and in that solids contents of the a-sulfofatty acid ester salts above 35% by weight are adjusted in the following aqueous working-up step.

2. A process as claimed in Claim 1, characterized in that the afterreaction with the alcohols or alkoxylation products thereof is carried out at temperatures above 75°C and preferably at temperatures of from 80 to 100°C and in that the hydroxyl-containing components are used in particular in substantially equimolar quantities, based on the $SO_3$ unused for the α-sulfonation.

3. A process as claimed in Claims 1 and 2, characterized in that paste solids contents of from about 40 to 65% by weight are adjusted in the following aqueous working-step comprising in particular bleaching and/or neutralization to the ester sulfonate salt.

4. A process as claimed in Claims 1 to 3, characterized in that the afterreaction is carried out with monofunctional alkanols or alkoxylation products thereof containing up to 30 carbon atoms in the alcohol residue and preferably up to 20 alkoxy groups in the molecule, $C_2$–$C_{22}$ alcohols and/or alkoxylation products thereof containing at least some EO-groups preferably being used.

5. A process as claimed in Claims 1 to 4, characterized in that the afterreaction is carried out with alkoxylation products of lower alcohols containing up to 6 carbon atoms and preferably from 2 to 4 carbon atoms and from 2 to 15 and preferably from 5 to 12 EO-units in the alkoxy group and/or with fatty alcohols or alkoxylation products thereof.

6. A process as claimed in Claims 1 to 5, characterized in that crude sulfonic acid containing no more than 80 mole% and preferably no more than 50 mole% $SO_3$ unused in the α-sulfonation, based in each case on α-sulfofatty acid ester, is subjected to the afterreaction, this crude sulfonic acid preferably having a degree of sulfonation of at least 90% and, more especially, of at least 95%.

7. A process as claimed in Claims 1 to 6, characterized in that the ester sulfonate pastes prepared have a disalt content of no more than about 10% by weight and preferably of no more than 5% by weight, based in each case on washing-active substance, and, at the same time, are preferably substantially free from unbound alcohol in troublesome amounts.

## Revendications

1. Procédé de préparation de pâtes aqueuses de sels d'esters d'acides gras alpha-sulfonés à activité de lavage qui, à de hautes teneurs en matières solides, sont également mobiles à des températures modérément élevées et, en particulier, aptes au pompage, moyennant une sulfonation d'esters alkyliques d'acides gras avec $SO_3$ dans des rapports molaires allant jusqu'à 1 : 2, en particulier, de 1 : 1,2 à 1,8, avec traitement ultérieur de l'acide sulfonique brut en milieu aqueux et avec formation de sel, caractérisé en ce que, avant le traitement avec un milieu aqueux, on soumet l'acide sulfonique brut à une réaction ultérieure avec au moins environ 0,5 équivalent molaire (calculé sur la fraction de $SO_3$ non consommée pour l'alpha-sulfonation) d'alcools monovalents et/ou de leurs produits d'alcoxylation à des températures supérieures à 70°C tandis que, lors du traitement aqueux ultérieur, on règle des teneurs en matières solides de plus de 35% en poids dans les sels d'esters d'acides gras alpha-sulfonés.

2. Procédé selon la revendication 1, caractérisé en ce qu'on effectue la réaction ultérieure avec les alcools ou leurs produits d'alcoxylation à des températures supérieures à 75°C, de préférence, à des températures se situant dans l'intervalle allant de 80 à 100°C tandis que, en l'occurrence, on utilise, en particulier, des quantités à peu près équimolaires des composants contenant des groupes hydroxyle (calculé sur la fraction de $SO_3$ non consommée pour l'alpha-sulfonation).

3. Procédé selon les revendications 1 et 2, caractérisé en ce que, lors du traitement aqueux ultérieur (en particulier, le blanchiment et/ou la neutralisation en sel ester-sulfonate), on règle des teneurs en matières solides pâteuses d'environ 40 à 65% en poids.

4. Procédé selon les revendications 1 à 3, caractérisé en ce qu'on effectue la réaction ultérieure avec des alcanols monofonctionnels ou leurs produits d'alcoxylation contenant jusqu'à 30 atomes de carbone dans le radical alcool et, de préférence, jusqu'à 20 radicaux alcoxy dans la molécule, tout en travaillant, de préférence avec des alcools en $C_2$–$C_{22}$ et/ou leurs produits d'alcoxylation contenant au moins en partie des groupes EO.

5. Procédé selon les revendications 1 à 4, caractérisé en ce qu'on effectue la réaction ultérieure avec des produits d'alcoxylation d'alcools inférieurs contenant jusqu'à 6 atomes de carbone, de préférence, 2 à 4 atomes de carbone, ainsi que 2 à 15, de préférence, 5 à 12 motifs EO dans le radical alcoxy et/ou avec des alcools gras ou leurs produits d'alcoxylation.

6. Procédé selon les revendications 1 à 5, caractérisé en ce qu'on soumet, à la réaction ultérieure, des acides sulfoniques bruts dont la fraction de $SO_3$ non consommée lors de l'alpha-sulfonation ne dépasse pas plus de 80% molaires, de préférence, pas plus de 50% molaires (calculé chaque fois sur l'ester d'acide gras alpha-sulfoné), ces acides sulfoniques bruts ayant, de préférence, un degré de sulfonation d'au moins 90%, en particulier, d'au moins 95%.

7. Procédé selon les revendications 1 à 6, caractérisé en ce qu'on prépare des pâtes d'esters-sulfonates ayant une teneur en disels ne dépassant pas environ 10% en poids, de préférence, ne dépassant pas 5% en poids (calculé chaque fois sur la substance active de lavage) qui de préférence et simultanément, sont essentiellement exemptes d'alcool non fixé en quantités gênantes.

Figur 1

Figur 2

Figur 3